# EUROPEAN PATENT APPLICATION

(11) **EP 4 235 162 A1**
(43) Date of publication of application: **30.08.2023**
(21) Application number: 22736134.2
(22) Date of filing: 21.02.2022
(51) Int. Cl.: G01N 27/26

(54) **ELECTROCHEMICAL TEST BASE AND DEVICE FOR ACTIVATED CLOTTING TIME**

(30) Priority: 13.01.2022 CN 202210039227
(71) Applicant: Lansion Biotechnology Co., Ltd, Nanjing, Jiangsu 211100 (CN)
(72) Inventor: XU, Xingshang, Nanjing, Jiangsu 211100 (CN); CHEN, Jeffery, Nanjing, Jiangsu 211100 (CN); WANG, Long, Nanjing, Jiangsu 211100 (CN); YANG, Shen, Nanjing, Jiangsu 211100 (CN)
(74) Representative: ZHAOffice SPRL
(86) International application number: PCT/CN2022/077011
(87) International publication number: WO 2023/133968

(57) **Abstract**

The present invention discloses an electrochemical test base and apparatus for activated clotting time (ACT). The base includes a chip inlet. A magnetic field change structure is disposed above or below a position of the chip inlet. An ACT test chip is inserted from the chip inlet. The magnetic field change structure uniformly mixes a blood sample and a reactive reagent in the ACT test chip. The ACT test chip added with the blood sample is inserted from the chip inlet of the base. The magnetic field change structure is disposed above or below the position of the chip inlet, and applies a magnetic field to the chip. The magnetic field change structure can generate a changing magnetic field. An inertial magnetic rotating rod in a chip test cavity uniformly mixes the blood sample and the reactive reagent in the chip for thorough reaction, to ensure accurate test results, thereby resolving problems of the hysteresis and poor stability of test results.

## Description

### TECHNICAL FIELD

The present invention belongs to the technical field of fast test in vitro diagnostic equipment, and in particular, to an electrochemical test base and apparatus for activated clotting time (ACT).

### BACKGROUND

For conventional coagulation test items, a patient's plasma needs to be drawn intravenously into a sodium citrate anticoagulation tube and is centrifuged as required, and then the upper layer of the plasma is transferred to a large blood coagulation analyzer for test. It takes a long time to obtain test results. In addition, a patient needs to go to the hospital. Especially, for a patient with thrombosis who needs to take anticoagulants for a long time, frequent visits to the hospital for review are time-consuming and labor-intensive.

Chinese Patent Document CN209690317U discloses a multi-indicator coagulation item test system. The multi-indicator coagulation item test system includes a housing, a test heating module, a battery module, a touch display screen, and a motherboard. The housing includes an upper casing and a lower casing. The housing is enclosed by the upper casing and the lower casing butted together to form an enclosed space. The motherboard is disposed between the upper casing and the lower casing and is disposed in the enclosed space. The touch display screen is disposed on the upper surface of the upper casing. The touch display screen is connected to the motherboard. The test heating module is disposed in the enclosed space, and is disposed at the front end of the lower casing and is connected to the motherboard. The battery module is disposed at the bottom of the end of the lower casing, and the battery module is connected to the motherboard. The front end of the housing is provided with a test inlet for inserting and placing a test card.

The multi-indicator coagulation item test system in the foregoing technical solution can perform instant test and can be operated without professional personnel. The multi-indicator coagulation item test system is convenient to operate and has high sensitivity and constant heating temperature. However, during actual use, electrical signals are prone to hysteresis, test results are inaccurate and unstable, and it is necessary to make improvements.

### SUMMARY

A technical problem to be resolved by the present invention is to provide an electrochemical test base for ACT, so that a blood sample and a reactive reagent in a chip can be uniformly mixed for thorough reaction, to ensure accurate test results, thereby resolving problems of the hysteresis and poor stability of test results.

The technical solutions used in the present invention to resolve the foregoing technical problem are as follows: The electrochemical test base for ACT includes a chip inlet, where a magnetic field change structure is disposed above or below a position of the chip inlet; and an ACT test chip is inserted from the chip inlet, and the magnetic field change structure uniformly mixes a blood sample and a reactive reagent in the ACT test chip.

The ACT test chip added with the blood sample is inserted from the chip inlet of the base. The magnetic field change structure is disposed above or below the position of the chip inlet, and applies a magnetic field to the chip. The magnetic field change structure can generate a changing magnetic field. An inertial magnetic rotating rod in a chip test cavity uniformly mixes the blood sample and the reactive reagent in the chip for thorough reaction, to ensure accurate test results, thereby resolving problems of the hysteresis and poor stability of test results.

Preferably, the magnetic field change structure includes a magnet fixing block, and at least two magnets are placed at different positions on the magnet fixing block; and the magnet fixing block is driven by a rotation assembly to rotate to cause the magnetic field change structure to generate a changing magnetic field.

The at least two magnets are placed at different positions on the magnet fixing block, so that the positions of the magnets change when the magnet fixing block rotates, and the magnets can generate a changing magnetic field. The rotation assembly is used for driving the magnet fixing block to rotate.

Preferably, the rotation assembly includes a rotation control motor, a rotation driving pulley, a belt, a rotation driven pulley, and a rotation shaft; and the rotation shaft is connected to the magnet fixing block, the rotation driving pulley is driven by the rotation control motor to rotate, the belt drives the rotation driven pulley to rotate, and the rotation driven pulley is connected to the rotation shaft.

Preferably, a heating sheet is disposed below the chip inlet, and the heating sheet is used for constant temperature reaction of the blood sample and the reactive reagent in the chip.

The heating sheet is used for turning on a heating function of the base, to keep constant temperature reaction in the ACT test chip at 37°C. A temperature control sensor is provided on the heating sheet. A reaction environment of the ACT test chip is heated from the beginning, to fully ensure a reaction condition of the ACT test chip, and the test of a single indicator ensures higher accuracy of a result.

Preferably, the base further includes a base upper casing and a base lower casing, where a concave groove is formed after the base upper casing and the base lower casing are assembled; and a circuit board and a test start button are further disposed in the electrochemical test base for ACT.

The concave groove is used for placing a blood coagulation analyzer. The heating function of the heating sheet is turned on when the test start button is pressed.

Preferably, the ACT test chip includes a chip body and a chip housing; a front end of the chip body is a chip test end, the chip body is further provided with a chip feed end, and the chip feed end is far away from the chip test end; and the chip feed end is disposed in the chip housing.

Blood in the ACT test chip gradually clots. An electrical signal is transmitted through an electrode during reaction. The chip test end is connected to the blood coagulation analyzer to obtain a test result of ACT. The test result is displayed on a display screen of the blood coagulation analyzer, or the test result may be printed out. The chip feed end is disposed in the chip housing, and the chip housing supports the ACT test chip.

Preferably, the chip housing includes a chip housing upper casing and a chip housing lower casing; a chip feed port is disposed at the chip housing upper casing, and the blood sample enters the chip body through the chip feed port; and the chip housing upper casing and the chip housing lower casing are assembled to clamp the chip feed end.

Preferably, a chip housing holding position, a chip feed cap, and a housing concave position are disposed at the chip housing upper casing, and the chip feed cap is used for opening or closing the chip feed port.

The chip housing holding position facilitates the holding of the chip and feed a sample, and is ergonomically designed. The chip feed cap is opened to allow the addition of a to-be-tested sample. The blood sample enters the test cavity of the ACT test chip to react. The chip feed cap can prevent reactive materials in a test cavity of the chip body from falling out. The housing concave position and the entire chip housing together provide a supporting force for the chip body to stably connect the chip body and the housing.

Preferably, the chip body is provided with a chip body upper layer and a chip body middle layer; the chip body upper layer is provided with an upper-layer feed port through hole, and the chip body middle layer is provided with a middle-layer feed port through hole; and the upper-layer feed port through hole is larger than the middle-layer feed port through hole, the chip body middle layer is a double sided adhesive layer, and when the chip body upper layer and the chip body middle layer are bonded, a part of the chip body middle layer around the middle-layer feed port through hole is in contact with an opposite surface of the chip housing upper casing to bond the chip body and the chip housing upper casing.

Structurally, the upper-layer feed port through hole is larger than the middle-layer feed port through hole. Therefore, a part of the chip body middle layer passing through the upper-layer feed port through hole is bonded to the chip housing upper casing, to bond the chip body and the chip housing upper casing together. The chip body middle layer is a double sided adhesive layer, and bonds together an upper chip layer and a lower chip layer that are adjacent to the chip body middle layer.

Preferably, the chip body is further provided with a chip body lower layer, and the chip feed port, the upper-layer feed port through hole, the middle-layer feed port through hole, and the chip body lower layer together form a chip test cavity.

Preferably, an inertial magnetic rotating rod is disposed in the chip test cavity, and an ACT test solidified reagent is further placed in the chip test cavity.

The changing magnetic field drives the inertial magnetic rotating rod to rotate to thoroughly and uniformly mix the blood sample and the ACT test solidified reagent in the chip test cavity to react.

Preferably, the inertial magnetic rotating rod is a nickel rod, and the nickel rod is fixed at the chip body lower layer in the chip test cavity in a soluble bonding manner.

The nickel rod is fixed in a soluble bonding manner and is prevented from falling out easily. After the blood sample is added, an adhesive bonding the nickel rod can be dissolved, making the nickel rod drifting in a reaction system.

Preferably, a positioning post is disposed at the chip housing lower casing, and the positioning post is clamped with a positioning groove provided in the chip housing upper casing, to fix the chip feed end inside the chip housing.

Preferably, the chip test end includes a test electrode located on the chip body lower layer, and the test electrode is connected to the chip test cavity; and an electrical signal is transmitted through the test electrode during reaction in the chip test cavity.

Preferably, the inertial magnetic rotating rod has a diameter of 0.5 mm to 1.5 mm and a length of 5 mm to 7 mm; and a feed amount of the chip test cavity is 50 µL to 100 µL.

Preferably, a limit switch is disposed at a position near the chip inlet, and the limit switch is used for signal transmission when the ACT test chip is inserted in position.

Another technical problem to be resolved by the present invention is to provide an electrochemical test apparatus for ACT, including the electrochemical test base for ACT, and further including a blood coagulation analyzer, where an ACT test chip is inserted from a chip inlet of the electrochemical test base for ACT and enters a feed port in the blood coagulation analyzer.

Preferably, the blood coagulation analyzer is located in the electrochemical test base for ACT.

### BRIEF DESCRIPTION OF THE DRAWINGS

The accompanying drawings and the implementations of the present invention are combined for further description below.
FIG 1 is a schematic structural diagram of an electrochemical test base for ACT according to the present invention;
FIG 2 is an internal schematic structural diagram of the electrochemical test base for ACT in FIG 1;
FIG 3 is an internal schematic structural diagram of the electrochemical test base for ACT (assembled with an ACT test chip) in FIG 1;
FIG 4 is an internal schematic structural sectional view of the electrochemical test base for ACT in FIG 1;
FIG 5 is a schematic structural bottom view of FIG 2;
FIG 6 is a schematic structural diagram of assembling a rotation assembly and a magnetic field change structure;
FIG 7 is a schematic structural diagram of an ACT test chip;
FIG 8 is a schematic structural sectional view of the ACT test chip in FIG 7;
FIG 9 is a schematic structural diagram of the bottom of the ACT test chip (without a chip housing lower casing) in FIG 7;
FIG 10 is a schematic structural diagram of an open state of a chip feed cap of an ACT test chip;
FIG 11 is an overall schematic structural front view of FIG 10;
FIG 12 is a schematic structural rear view of FIG 11;
FIG 13 is a structural exploded view of the ACT test chip in FIG 7;
FIG 14 is an overall schematic structural front view of a chip housing;
FIG 15 is a schematic structural rear view of FIG 14;
FIG 16 is a schematic structural diagram of a chip housing lower casing;
FIG 17 is a schematic structural perspective front view of an ACT test chip;
FIG 18 is an overall schematic structural diagram of an electrochemical test apparatus for ACT according to the present invention;
FIG 19 is a structural diagram of an electrochemical test apparatus for ACT (without a top cover removed) according to the present invention;
FIG 20 is a chart of correlation analysis of test results of an electrochemical test apparatus for ACT and a contrast instrument according to the present invention; and
FIG 21 is a chart of heparin sensitivity analysis (added correlation analysis of a standard heparin concentration and an ACT test value of the standard heparin concentration) of an electrochemical test apparatus for ACT according to the present invention.

Where: 1-electrochemical test base for ACT, 101-chip inlet, 102-magnetic field change structure, 10201-magnet fixing block, 10202-magnet; 103-rotation assembly, 10301-rotation control motor, 10302-rotation driving pulley, 10303-belt, 10304-rotation driven pulley, 10305-rotation shaft, 104-heating sheet, 105-base upper casing, 106-base lower casing, 107-concave groove, 108-circuit board, 109-test start button, 1010-limit switch; 2-ACT test chip, 201-chip body, 20101-chip test end, 20102-chip feed end, 20103-chip body upper layer, 20104-chip body middle layer, 20105-upper-layer feed port through hole, 20106-middle-layer feed port through hole, 20107-part of the chip body middle layer around the middle-layer feed port through hole, 20108-chip body lower layer, 20109-chip test cavity, 201010-test electrode, 202-chip housing, 20201-chip housing upper casing, 20202-chip housing lower casing, 20203-chip feed port, 20204-chip housing holding position, 20205-chip feed cap, 20206-housing concave position, 20207-opposite surface of the chip housing upper casing, 20208-positioning post, 20209-positioning groove, 203-inertial magnetic rotating rod; 3-electrochemical test apparatus for ACT; 4-blood coagulation analyzer, and 401-feed port.

### DETAILED DESCRIPTION OF THE INVENTION

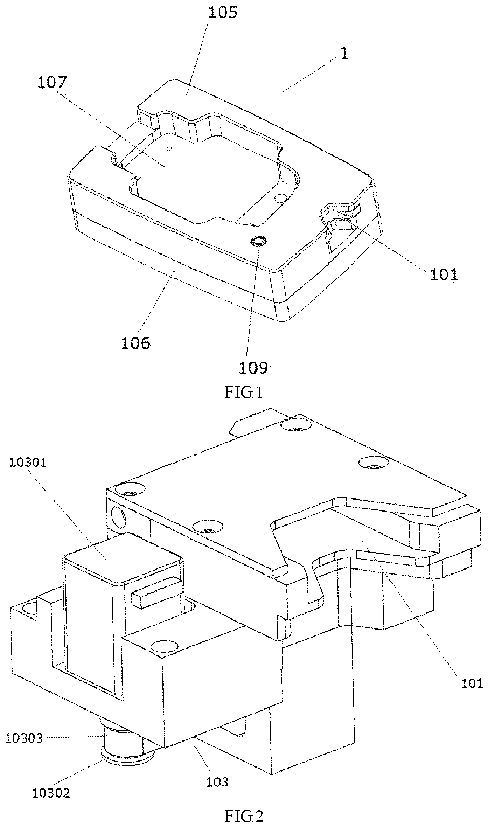

FIG 1 shows an electrochemical test base 1 for ACT in this embodiment, including a chip inlet 101. A magnetic field change structure 102 is disposed above or below a position of the chip inlet 101. An ACT test chip 2 is inserted from the chip inlet 101. The magnetic field change structure 102 uniformly mixes a blood sample and a reactive reagent in the ACT test chip 2 by using an inertial magnetic rotating rod 203.

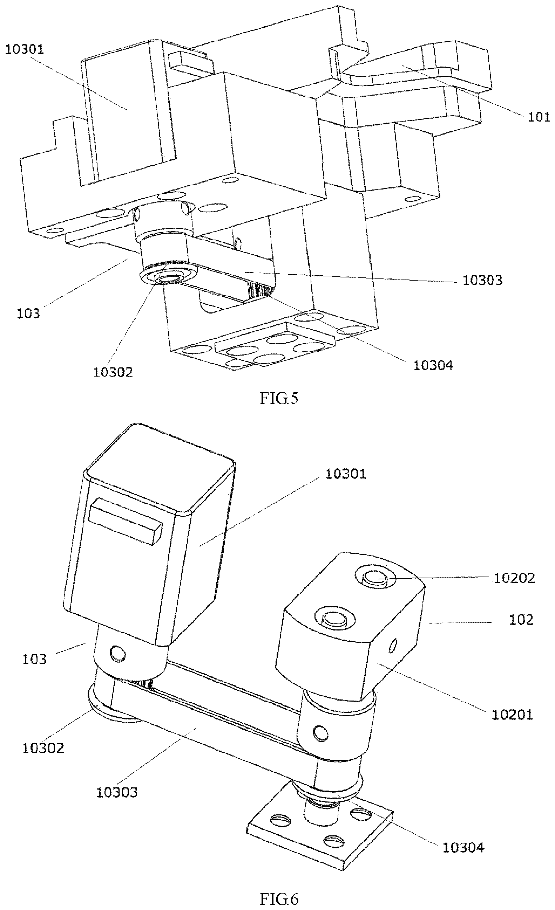

The magnetic field change structure 102 includes a magnet fixing block 10201. At least two magnets 10202 are placed at different positions on the magnet fixing block 10201. In this embodiment, as shown in FIG 6, two magnets are placed at two ends in a length direction. The magnet fixing block 10201 is driven by a rotation assembly 103 to rotate to cause the magnetic field change structure 102 to generate a changing magnetic field.

The rotation assembly 103 includes a rotation control motor 10301, a rotation driving pulley 10302, a belt 10303, a rotation driven pulley 10304, and a rotation shaft 10305. The rotation shaft 10305 is connected to the magnet fixing block 10201. The rotation driving pulley 10302 is driven by the rotation control motor 10301 to rotate. The belt 10303 drives the rotation driven pulley 10304 to rotate. The rotation driven pulley 10304 is connected to the rotation shaft 10305.

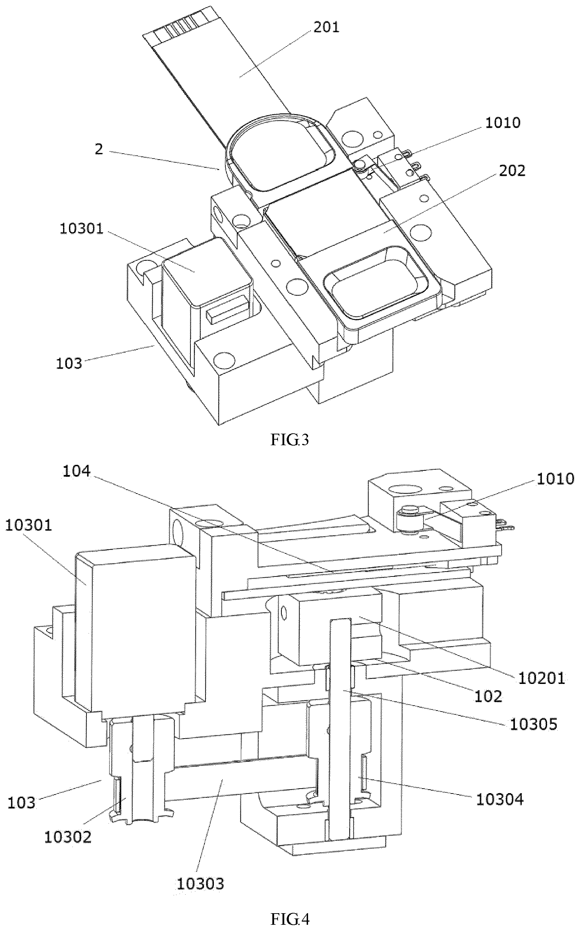

A heating sheet 104 is disposed below the chip inlet 101. As shown in FIG 4, the heating sheet 104 is used for constant temperature reaction of the blood sample and the reactive reagent in the ACT test chip 2 at 37°C.

The base further includes a base upper casing 105 and a base lower casing 106. A concave groove 107 is formed after the base upper casing 105 and the base lower casing 106 are assembled. A circuit board 108 and a test start button 109 are further disposed in the electrochemical test base 1 for ACT.

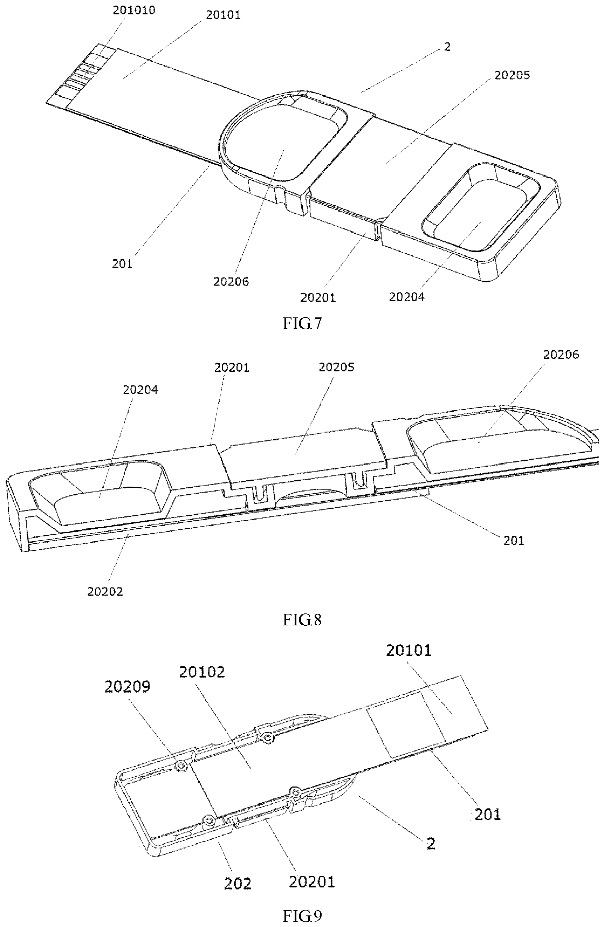

As shown in FIG 7 to FIG 17, the ACT test chip 2 includes a chip body 201 and a chip housing 202. A front end of the chip body 201 is a chip test end 20101. The chip body 201 is further provided with a chip feed end 20102. The chip feed end 20102 is far away from the chip test end 20101. The chip feed end 20102 is disposed in the chip housing 202.

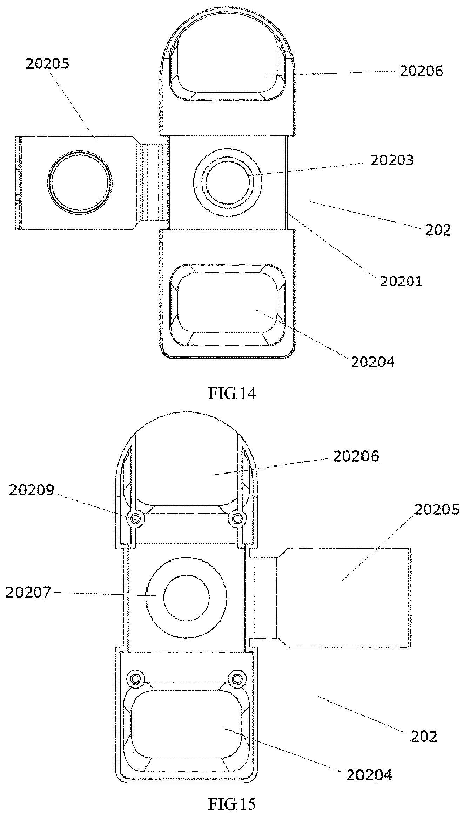

As shown in FIG 14 to FIG 16, the chip housing 202 includes a chip housing upper casing 20201 and a chip housing lower casing 20202. A chip feed port 20203 is disposed at the chip housing upper casing 20201. The blood sample enters the chip body 201 through the chip feed port 20203. The chip housing upper casing 20201 and the chip housing lower casing 20202 are assembled to clamp the chip feed end 20102.

A chip housing holding position 20204, a chip feed cap 20205, and a housing concave position 20206 are disposed at the chip housing upper casing 20201. The chip feed cap 20205 is used for opening or closing the chip feed port 20203.

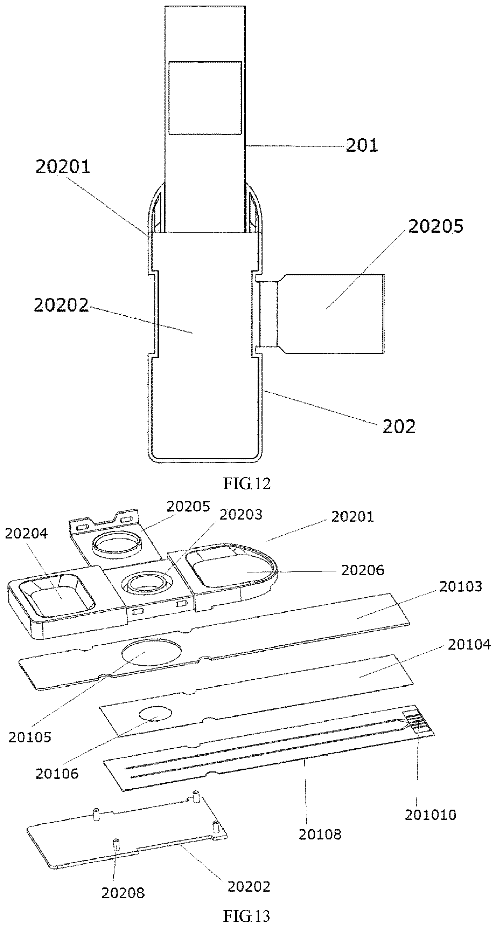

As shown in FIG 13, the chip body 201 is provided with a chip body upper layer 20103 and a chip body middle layer 20104. The chip body upper layer 20103 is provided with an upper-layer feed port through hole 20105. The chip body middle layer 20104 is provided with a middle-layer feed port through hole 20106. The upper-layer feed port through hole 20105 is larger than the middle-layer feed port through hole 20106. The chip body middle layer 20104 is a double sided adhesive layer (that is, both an upper surface and a lower surface are provided with an adhesive). When the chip body upper layer 20103 and the chip body middle layer 20104 are bonded, a part of the chip body middle layer 20107 around the middle-layer feed port through hole (that is, a part of the chip body passing through the upper-layer feed port through hole 20105) is in contact with an opposite surface of the chip housing upper casing 20201 to bond the chip body 201 and the chip housing upper casing 20201, as shown in FIG 15.

The chip body 201 is further provided with a chip body lower layer 20108, and the chip feed port 20203, the upper-layer feed port through hole 20105, the middle-layer feed port through hole 20106, and the chip body lower layer 20108 together form a chip test cavity 20109.

The inertial magnetic rotating rod 203 is disposed in the chip test cavity 20109. An ACT test solidified reagent is further placed in the chip test cavity 20109. The inertial magnetic rotating rod is a nickel rod. The nickel rod is fixed at the chip body lower layer 20108 in the chip test cavity 20109 in a soluble bonding manner.

The magnets 10202 are fixedly disposed on the magnet fixing block 10201. The magnet fixing block 10201 rotates. The magnet fixing block 10201 is disposed at a bottom surface of the chip test cavity 20109 of the ACT test chip 2. The nickel rod (made of an inertial magnetic material, that is, the inertial magnetic rotating rod 203) is disposed in the chip test cavity 20109 and has a length of 5 mm to 7 mm and a diameter of 0.5 mm to 1.5 mm, and preferably has a length of 6 mm and a diameter of 1 mm. Under the action of the magnet fixing block 10201, the nickel rod moves in the chip test cavity 20109, to thoroughly and uniformly mix the blood sample and a solidified reagent in the test cavity to react.

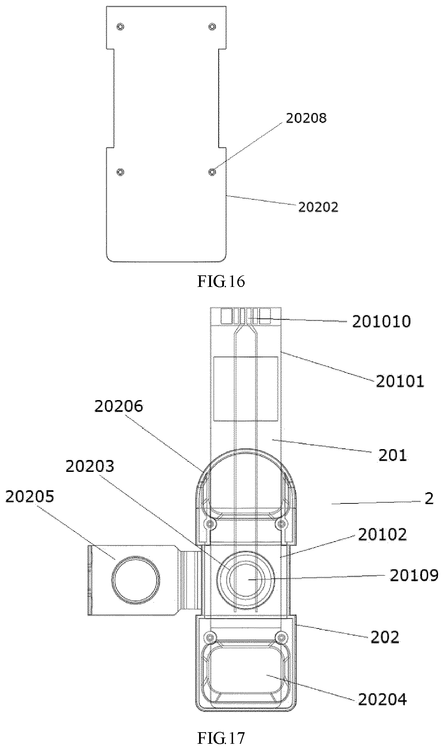

A positioning post 20208 is disposed at the chip housing lower casing 20102, as shown in FIG 13 and FIG 16. The positioning post 20208 is clamped with a positioning groove 20209 provided in the chip housing upper casing 20201, to fix the chip feed end 20102 inside the chip housing 202.

Preferably, the chip test end 20101 includes a test electrode 201010 located on the chip body lower layer 20108. As shown in FIG 7, FIG 10, FIG 11, FIG 13, and FIG 17, the test electrode 201010 is connected to the chip test cavity 20109.

It needs to be noted that each of the ACT test chip 2 and the chip body 201 in this embodiment has a three-layer structure. The chip body middle layer 20104 is a double sided adhesive layer, and is separately bonded to the chip body upper layer 20103 and the chip body lower layer 20108.

The characteristics of the ACT test chip 2 (including a housing upper casing and a housing lower casing) in the present invention are as follows: (1) There are a total of five layers. One chip test cavity 20109 coordinates with the corresponding test electrode 201010 to perform test. A feed flow channel in an existing chip design is canceled, and a feed process is not required. After a sample is added to the chip test cavity 20109 (the test cavity is used as a reaction cavity), a test process is started. (2) The inertial magnetic rotating rod 203 (a high-purity nickel rod) fitting the test electrode 201010 is used in combination to implement real-time test of ACT, and a test result is provided as soon as a blood coagulation reaction ends. (3) The corresponding electrochemical test base for ACT (having an incubation function) is used in combination, ACT can be tested by using a fitting blood coagulation analyzer, and no other complex test equipment is required. (4) The chip feed cap 20205 and the chip housing upper casing 20201 are combined, and the ACT test chip 2 is integrally formed, to ensure that sample test causes no biological contamination to the external environment. (5) A magnetic and inertial material rod is preplaced in the chip test cavity 20109 of the ACT test chip 2, and is preferably a nickel rod. The diameter (0.5 mm to 1.5 mm) of the nickel rod is preferably 1 mm, and the length (5 mm to 7 mm) is preferably 6 mm, to thoroughly and uniformly mix reactants, thereby ensuring the accuracy of test results. (6) A feed amount of the chip test cavity 20109 of the ACT test chip 2 is 50 µL to 100 µL, adequate compatibility with application scenarios is provided, thereby meeting requirements of in vitro diagnosis.

A limit switch 1010 is disposed at a position near the chip inlet 101, as shown in FIG 4. The limit switch 1010 is used for signal transmission when the ACT test chip 2 is inserted in position.

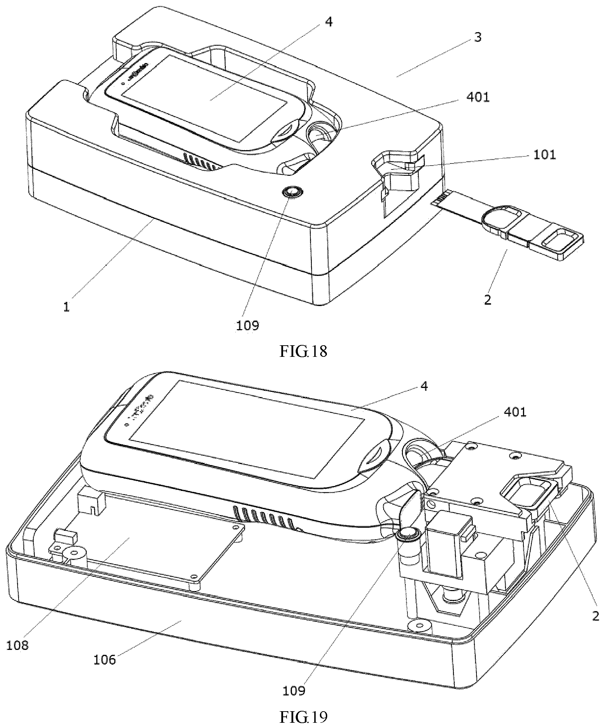

As shown in FIG 18 and FIG 19, an electrochemical test apparatus 3 for ACT includes the electrochemical test base 1 for ACT, and further includes a blood coagulation analyzer 4. An ACT test chip 2 is inserted from a chip inlet 101 of the electrochemical test base 1 for ACT and enters a feed port 401 in the blood coagulation analyzer 4. The blood coagulation analyzer 4 is located in the electrochemical test base 1 for ACT.

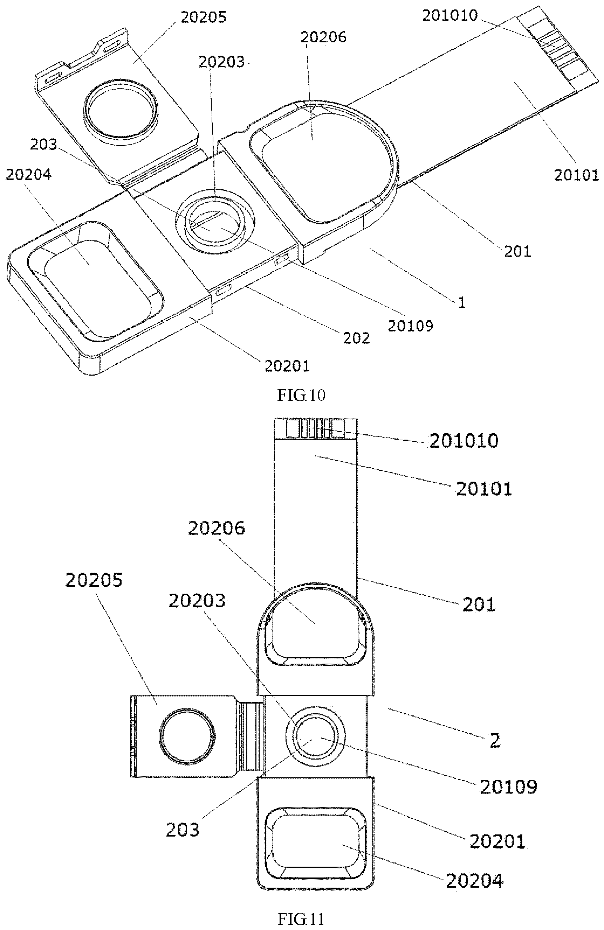

During use, as shown in FIG 19, the test start button 109 of the electrochemical test base 1 for ACT is pressed, and a heating function of the electrochemical test base 1 for ACT is turned on. The ACT test chip 2 added with the blood sample is inserted from the chip inlet 101. The chip test end 20101 enters the blood coagulation analyzer 4 from the feed port 401 of the blood coagulation analyzer 4 (is inserted in the concave groove 107). The blood sample in the chip test cavity 20109 of the ACT test chip 2 and the reactive reagent are stirred by the nickel rod of the built-in inertial magnetic rotating rod 203 (because the inertial magnetic rotating rod is very small, on a micrometer level or a millimeter level, and FIG 10 and FIG 11 only show the position of the inertial magnetic rotating rod) to thoroughly and uniformly react. Blood gradually clots. An electrical signal is transmitted through the test electrode 201010 during reaction. Finally, the blood coagulation analyzer 4 calculates a coagulation time point to obtain a test result of ACT. The test result is displayed on a display screen of the blood coagulation analyzer 4, or the test result may be printed out.

A chip performance experiment was performed on the ACT test chip 2. A test method was as follows: The ACT test chip 2 and the electrochemical test apparatus 3 for ACT in the present invention were used to perform ACT test on 50 normal healthy people. Everyone was tested once, and readings were recorded. Before testing, quality control was performed first. Test was then performed. Test data of 50 samples are shown in the following Table 1.

**Table 1**

| Test values of the samples 1 to 50 | | | | | | | | | |
|---|---|---|---|---|---|---|---|---|---|
| 103 | 118 | 113 | 110 | 113 | 118 | 118 | 117 | 120 | 114 |
| 119 | 104 | 120 | 108 | 106 | 108 | 116 | 113 | 118 | 107 |
| 117 | 118 | 100 | 110 | 103 | 104 | 103 | 101 | 109 | 102 |
| 110 | 119 | 118 | 104 | 104 | 115 | 100 | 107 | 102 | 117 |
| 114 | 112 | 107 | 105 | 112 | 115 | 105 | 117 | 115 | 114 |

According to the foregoing test data, a reference value range of a chip is shown in the following Table 2:

**Table 2**

| | | | | |
|---|---|---|---|---|
| ACT test chip in the present invention | N | Mean | 2SD | Reference range |
| | 50 | 111 | 12 | 99s to 123s |

The reference value range of a chip is 99s to 123s. Every laboratory may establish its own reference range.

A correlation experiment was further performed on the ACT test chip 2 in this embodiment. A test method is as follows: A contrast instrument, that is, Helena (USA) (Model: Actalyke MINI II) and the present invention were separately used to test 100 different people. Everyone was tested once, and readings were recorded. Before testing, quality control was performed first. Test was then performed. Test data were shown in the following Table 3.

**Table 3**

| Sequence number | Target value (s)-Helena | Test value (s) of the present invention | Sequence number | Target value (s)-Helena | Test value (s) of the present invention |
|---|---|---|---|---|---|
| 1 | 101 | 97 | 51 | 520 | 507 |
| 2 | 114 | 101 | 52 | 530 | 518 |
| 3 | 120 | 105 | 53 | 534 | 562 |
| 4 | 120 | 110 | 54 | 535 | 539 |
| 5 | 125 | 113 | 55 | 542 | 513 |
| 6 | 126 | 119 | 56 | 547 | 556 |
| 7 | 138 | 121 | 57 | 556 | 592 |
| 8 | 142 | 150 | 58 | 557 | 528 |
| 9 | 142 | 130 | 59 | 557 | 586 |
| 10 | 148 | 150 | 60 | 587 | 596 |
| 11 | 158 | 172 | 61 | 593 | 620 |
| 12 | 162 | 183 | 62 | 602 | 659 |
| 13 | 168 | 160 | 63 | 607 | 582 |
| 14 | 172 | 164 | 64 | 648 | 621 |
| 15 | 203 | 211 | 65 | 650 | 675 |
| 16 | 205 | 228 | 66 | 669 | 679 |
| 17 | 213 | 201 | 67 | 674 | 684 |
| 18 | 216 | 228 | 68 | 675 | 698 |
| 19 | 229 | 250 | 69 | 678 | 694 |
| 20 | 230 | 215 | 70 | 687 | 621 |
| 21 | 231 | 234 | 71 | 689 | 651 |
| 22 | 241 | 265 | 72 | 690 | 652 |
| 23 | 255 | 231 | 73 | 696 | 629 |
| 24 | 268 | 298 | 74 | 698 | 681 |
| 25 | 269 | 287 | 75 | 701 | 688 |
| 26 | 271 | 289 | 76 | 710 | 680 |
| 27 | 275 | 245 | 77 | 718 | 750 |
| 28 | 282 | 274 | 78 | 720 | 701 |
| 29 | 295 | 252 | 79 | 725 | 729 |
| 30 | 304 | 315 | 80 | 734 | 741 |
| 31 | 310 | 358 | 81 | 744 | 744 |
| 32 | 314 | 352 | 82 | 756 | 751 |
| 33 | 315 | 305 | 83 | 759 | 741 |
| 34 | 318 | 339 | 84 | 762 | 788 |
| 35 | 326 | 349 | 85 | 840 | 896 |
| 36 | 337 | 353 | 86 | 841 | 812 |
| 37 | 347 | 385 | 87 | 849 | 811 |
| 38 | 354 | 381 | 88 | 852 | 887 |
| 39 | 361 | 341 | 89 | 946 | 989 |
| 40 | 372 | 395 | 90 | 955 | 912 |
| 41 | 438 | 448 | 91 | 956 | 925 |
| 42 | 450 | 468 | 92 | 965 | 921 |
| 43 | 456 | 499 | 93 | 967 | 977 |
| 44 | 461 | 491 | 94 | 970 | 993 |
| 45 | 472 | 491 | 95 | 987 | 997 |
| 46 | 478 | 499 | 96 | 991 | 943 |
| 47 | 481 | 521 | 97 | 995 | 1001 |
| 48 | 483 | 468 | 98 | 996 | 941 |
| 49 | 494 | 521 | 99 | 1001 | 955 |
| 50 | 502 | 486 | 100 | 1001 | 1004 |

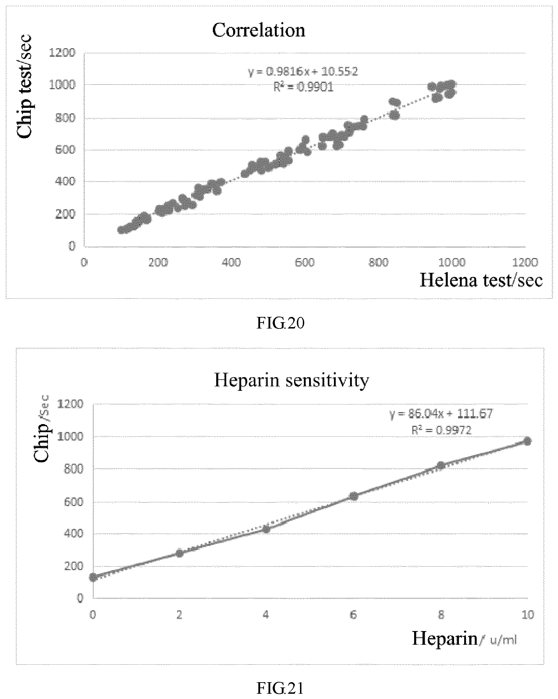

A correlation R² between the test results of the ACT test chip 2 in this embodiment and the target values of the contrast instrument Helena is 0.9901, indicating that the test performance of this embodiment is highly correlated to the contrast instrument, and test results are accurate and reliable, as shown in FIG 20.

A test method of a heparin sensitivity experiment is as follows: The ACT test chip 2 in the present invention was used to separately perform a test of heparin intervention with a plurality of concentrations on five people (six standard heparin concentrations for every person). A sample with each heparin concentration was tested once. An average value was calculated for samples with the same concentration, and data was recorded as shown in Table 4.

**Table 4**

| Heparin concentration (u/mL) | Test value (s) of the present invention | | | | | Average value (s) |
|---|---|---|---|---|---|---|
| | Person 1 | Person 2 | Person 3 | Person 4 | Person 5 | |
| 0 | 126 | 114 | 131 | 125 | 146 | 128.4 |
| 2 | 275 | 305 | 280 | 280 | 253 | 278.6 |
| 4 | 425 | 434 | 406 | 443 | 428 | 427.2 |
| 6 | 601 | 635 | 628 | 638 | 650 | 630.4 |
| 8 | 786 | 805 | 854 | 832 | 812 | 817.8 |
| 10 | 965 | 1008 | 905 | 1001 | 965 | 968.8 |

A curve result drawn from average values of ACT test data of heparin intervention with every concentration and heparin concentrations is shown in FIG 21. In the test of the present invention, R² is 0.9972, indicating that the test of ACT of the present invention is highly sensitive to heparin, and requirements of clinical test is satisfied.

The above shows and describes the basic principles, main features, and advantages of the present invention. A person skilled in the art should understand that the present invention is not limited by the above embodiments, and what is described in the above embodiments and the specification is only to describe the principle of the present invention. Without departing from the spirit and scope of the present invention, various changes and improvements may be made to the present invention, for example, changes may be made to the number of layers of the ACT test chip 2. All these changes and improvements fall within the scope that the present invention seeks to protect. The scope of protection of the present invention is defined by the appended claims and their equivalents.

## Claims

1. An electrochemical test base for activated clotting time (ACT), comprising a chip inlet, wherein a magnetic field change structure is disposed above or below a position of the chip inlet; and an ACT test chip is inserted from the chip inlet, and the magnetic field change structure uniformly mixes a blood sample and a reactive reagent in the ACT test chip.

2. The electrochemical test base for ACT according to claim 1, wherein the magnetic field change structure comprises a magnet fixing block, and at least two magnets are placed at different positions on the magnet fixing block; and the magnet fixing block is driven by a rotation assembly to rotate to cause the magnetic field change structure to generate a changing magnetic field.

3. The electrochemical test base for ACT according to claim 2, wherein the rotation assembly comprises a rotation control motor, a rotation driving pulley, a belt, a rotation driven pulley, and a rotation shaft; and the rotation shaft is connected to the magnet fixing block, the rotation driving pulley is driven by the rotation control motor to rotate, the belt drives the rotation driven pulley to rotate, and the rotation driven pulley is connected to the rotation shaft.

4. The electrochemical test base for ACT according to claim 1, wherein a heating sheet is disposed below the chip inlet, and the heating sheet is used for constant temperature reaction of the blood sample and the reactive reagent in the ACT test chip.

5. The electrochemical test base for ACT according to claim 1, further comprising a base upper casing and a base lower casing, wherein a concave groove is formed after the base upper casing and the base lower casing are assembled; and a circuit board and a test start button are further disposed in the electrochemical test base for ACT.

6. The electrochemical test base for ACT according to any one of claims 1 to 5, wherein the ACT test chip comprises a chip body and a chip housing; a front end of the chip body is a chip test end, the chip body is further provided with a chip feed end, and the chip feed end is far away from the chip test end; and the chip feed end is disposed in the chip housing.

7. The electrochemical test base for ACT according to claim 6, wherein the chip housing comprises a chip housing upper casing and a chip housing lower casing; a chip feed port is disposed at the chip housing upper casing, and the blood sample enters the chip body through the chip feed port; and the chip housing upper casing and the chip housing lower casing are assembled to clamp the chip feed end.

8. The electrochemical test base for ACT according to claim 6, wherein a chip housing holding position, a chip feed cap, and a housing concave position are disposed at the chip housing upper casing, and the chip feed cap is used for opening or closing the chip feed port.

9. The electrochemical test base for ACT according to claim 7, wherein the chip body is provided with a chip body upper layer and a chip body middle layer; the chip body upper layer is provided with an upper-layer feed port through hole, and the chip body middle layer is provided with a middle-layer feed port through hole; and the upper-layer feed port through hole is larger than the middle-layer feed port through hole, the chip body middle layer is a double sided adhesive layer, and when the chip body upper layer and the chip body middle layer are bonded, a part of the chip body middle layer around the middle-layer feed port through hole is in contact with an opposite surface of the chip housing upper casing to bond the chip body and the chip housing upper casing.

10. The electrochemical test base for ACT according to claim 9, wherein the chip body is further provided with a chip body lower layer, and the chip feed port, the upper-layer feed port through hole, the middle-layer feed port through hole, and the chip body lower layer together form a chip test cavity.

11. The electrochemical test base for ACT according to claim 10, wherein an inertial magnetic rotating rod is disposed in the chip test cavity, and an ACT test solidified reagent is further placed in the chip test cavity.

12. The electrochemical test base for ACT according to claim 11, wherein the inertial magnetic rotating rod is a nickel rod, and the nickel rod is fixed at the chip body lower layer in the chip test cavity in a soluble bonding manner.

13. The electrochemical test base for ACT according to claim 7, wherein a positioning post is disposed at the chip housing lower casing, and the positioning post is clamped with a positioning groove provided in the chip housing upper casing, to fix the chip feed end inside the chip housing.

14. The electrochemical test base for ACT according to claim 10, wherein the chip test end comprises a test electrode located on the chip body lower layer, and the test electrode is connected to the chip test cavity.

15. The electrochemical test base for ACT according to claim 6, wherein a limit switch is disposed at a position near the chip inlet, and the limit switch is used for signal transmission when the ACT test chip is inserted in position.

16. The electrochemical test base for ACT according to claim 10, wherein the inertial magnetic rotating rod has a diameter of 0.5 mm to 1.5 mm and a length of 5 mm to 7 mm; and a feed amount of the chip test cavity is 50 µL to 100 µL.

17. An electrochemical test apparatus for activated clotting time (ACT), comprising the electrochemical test base for ACT according to any one of claims 1 to 5, further comprising a blood coagulation analyzer, wherein an ACT test chip 2 is inserted from a chip inlet of the electrochemical test base for ACT and enters a feed port in the blood coagulation analyzer.

18. The electrochemical test apparatus for ACT according to claim 17, wherein the blood coagulation analyzer is located in the electrochemical test base for ACT.
